(19) **Europäisches Patentamt / European Patent Office / Office européen des brevets**

(11) **EP 1 463 475 B1**

(12) **EUROPEAN PATENT SPECIFICATION**

(45) Date of publication and mention
of the grant of the patent:
**11.03.2009 Bulletin 2009/11**

(51) Int Cl.:
***A61F 13/20*** (2006.01)

(21) Application number: **03729646.4**

(86) International application number:
**PCT/US2003/000852**

(22) Date of filing: **10.01.2003**

(87) International publication number:
**WO 2003/059231 (24.07.2003 Gazette 2003/30)**

(54) **ABSORBENT DEVICE WITH A LUBRICIOUS COVER**

ABSORBIERENDE VORRICHTUNG MIT GLEITFÄHIGER ABDECKUNG

DISPOSITIF ABSORBENT A ETUI LUBRIFIE

(84) Designated Contracting States:
**DE FR GB IT NL**

(30) Priority: **10.01.2002 US 43754**

(43) Date of publication of application:
**06.10.2004 Bulletin 2004/41**

(73) Proprietor: **McNEIL-PPC, Inc.
Skillman,
New Jersey 08558 (US)**

(72) Inventors:
• **NGUYEN, Hien, V
East Windsor, NJ 08520 (US)**
• **PIERSON, Linda, M
Stockton, NJ 08559 (US)**

• **SERBIAK, Paul
Yardley, PA 19067 (US)**
• **YANG, Ching-Yun, M
Princeton Junction, NJ 08550 (US)**

(74) Representative: **Metten, Karl-Heinz et al
Forrester & Boehmert,
Pettenkoferstrasse 20-22
80336 München (DE)**

(56) References cited:
**WO-A-99/27878        WO-A-99/32061
DE-A- 3 609 852        GB-A- 975 872
US-A- 3 390 671        US-A- 5 364 383
US-A- 5 731 083**

Note: Within nine months of the publication of the mention of the grant of the European patent in the European Patent Bulletin, any person may give notice to the European Patent Office of opposition to that patent, in accordance with the Implementing Regulations. Notice of opposition shall not be deemed to have been filed until the opposition fee has been paid. (Art. 99(1) European Patent Convention).

**Description**

**Field of the Invention**

The invention relates to novel absorbent tampons such as catamenial tampons. More particularly, the present invention relates to absorbent tampons having a cover with particular fluid retention properties. The cover incorporates modified cellulosic fibers having carboxyalkyl substituted.

**Background of the Invention**

Tampons have been used for internal absorption of body fluids for many years, especially for catamenial purposes. Improvements to materials and manufacturing methods have resulted in improved absorbencies.

US Pat. No. 3,005,456 (Graham) purports to disclose a catamenial device made from regnenerated cellulosic fibers that have a degree of carboxyalkyl substitution. The amount of substitution affected the absorbency of the catamenial device. This device, however, did not have a cover or wrapper to prevent fibers from sloughing from the absorbent device and does not address the ease of insertion or withdrawal of the device.

US Pat. No. 3,371,666 (Lewing) purports to disclose an absorbent device having a pad of cotton with water soluble, non-fibrous, carboxymethyl cellulose (CMC) having a degree of substitution above 0.35 dispersed within the pad in conjunction with an agglomeration-inhibiting means. The agglomeration-inhibiting means may be in the form of a thin film that is placed between the layers of cotton. By placing strips of the CMC film between layers of cotton and compressing, the absorbency of the prepared tampon could be increased. This device also lacks a cover or wrapper to prevent fibers from sloughing from the Absorbent device and does not address the ease of insertion or withdrawal of the device.

US Pat. No. 5,731,083 (Bahia et al.) purports to disclose a CMC solvent-spun fiber useful for absorbent articles such as diapers, sanitary napkins, tampons and absorbent wipes. The CMC solvent-spun fiber has a degree of substitution of at least 0.1 carboxymethyl group per glucose unit and is derived from solvent-spun cellulose fiber. The CMC solvent-spun fiber has an absorbency of at least 8 grams 0.9% saline solution per gram of fiber and a tenacity of at least 10 cN/tex. The fiber swells on contact with water. This disclosure suggests that the fibers may be blended with other absorbent fibers for use in an absorbent product.

Additionally, fluid permeable covers have been added to tampons for a variety of reasons: prevention of sloughing of individual fibers from the absorbent core during use, as an insertion aid to provide a surface of lessen friction and as a withdrawal aid. Conventional, absorbent tampons tend to wipe the vaginal tissue dry during insertion and in doing so, may cause undesirable irritation to the mucous membranes that line the vagina. During tampon withdrawal, negative or suction pressure may develop along the surface of the tampon that tends to make the vaginal walls adhere to the outer surface and makes withdrawal uncomfortable. The problems of vaginal irritation associated with removal may be progressively increased as tampon absorbency or volume increase.

Many efforts have been made to overcome these difficulties. Such efforts include coating the tip or leading edge of the tampon itself with lubricants such as petroleum jelly, emulsified mineral oil, soaps, surgical gel, and the like, or enclosing the tip of the tampon in a water-soluble film comprised of materials such as methylcellulose, gelatin, dextrin, glucose, polyvinyl alcohol, sodium alginate, etc.

Examples of various efforts to increase absorbency and improve the ease of insertion and removal can be found in the following references:

US Pat. No. 3,683,912 (Olson et al.) purports to disclose an absorbent tampon having a fluid pervious layer of polypropylene fibers disposed on its outer surface to ease insertion. The use of these substantially hydrophobic fibers does not provide a substantially wet or lubricious surface.

WO 90/02542 (Snider) purports to disclose a tampon having a coating of beeswax that reduces the release of fibers. The beeswax coating may contain an antigermicide, which purported to allow for higher absorbency with a reduced safety risk. The beeswax must be heated prior to application to the tampon. The beeswax may provide lubrication for ease of tampon removal, however, it may also reduce the tampon's ability to absorb fluid.

US Pat. No. 4,056,103 (Kaczmarzyk) purports to disclose a tampon having a highly absorbent core containing superabsorbent enclosed in a fluid-pervious wrapper. The wrapper structure is capable of containing a minor amount of the absorbed fluid sufficient to maintain the surface fibers in a soft, lubricous condition. This is apparently intended to successfully compete with the strongly absorbent superabsorbent materials in the absorbent core to retain fluid in the wrapper. One wrapper material disclosed contains a crosslinked carboxymethylcellulose superabsorbent fiber. It is purported that the wrapper eases of removal of the tampon. The highly absorbent core containing superabsorbent can extract fluid from vagina wall and causes discomfort during tampon removal.

WO 99/32061 A1 discloses a multilayered tampon cover for an absorbent tampon which substantially encloses an absorbent structure of said tampon. The cover comprises an outer layer capable of retaining liquid in an timer layer disposed between the outer layer and the absorbent structure. The inner layer is purported to create a controlled interruption of a fluid flow between the outer layer and the absorbent structure whereby the outer layer retains sufficient liquid to minimize vaginal wall drying prior to saturation of the absorbent structure. The controlled interruption can be achieved by creating a porosity gradient between the outer layer and the inner layer or by using a relatively more hydrophilic outer layer and a relatively less hydrophilic inner layer.

In GB 975,872 a medical tampon for the treatment of cavities of the human body is disclosed the outer surface of which is coated with a thin lubricating layer formed of a mucilaginous substance capable of being transformed into a slippery mucilage upon contact with moisture. Suitable mucilaginous substances comprise a gelatin, agar-agar, guar gum, alginates and carboxymethyl cellulose.

US 3,390,671 discloses a tubular tampon applicator the outer surface of which has been coated with a thin water soluble polymer which becomes slippery upon and by virtue of vaginal moistening. Suitable polymeric substances include polyvinyl alcohols, polyvinylpyrrolidone, polyethylene oxide, polyglycols, polyether film, alkali metal salts of polyacrylic acid, alkali metal salts of alginic acid, polysaccharides such as modified starch, sodium carboxymethyl celluloses and hydroxyethyl celluloses.

The abode examples seek to improve absorbencies by various ways. None, however, provide a lubricious tampon cover, solve the problems associated with ease removal, and offer comfortable tampons of appropriate absorbency All previous methods fail to provide easy removal of tampons with appropriate absorbency without sacrificing critical other properties. Thus, there is still a need for an absorbent tampon that is comfortable to remove.

Summary of the Invention

5 The present invention relates to an absorbent tampon, e.g., a vaginal tampon, according to claim 1. Said tampon has an absorbent structure including an absorbent component of absorbent material that has a Centrifuge Retention of distilled water of less than about 10 g/g. An outer layer substantially covers the absorbent structure. The outer layer has a different composition than the absorbent structure and a Centrifuge Retention of distilled water of at least about 1 g/g. The outer layer has modified cellulosic fibers that have carboxyalkyl substituted regions. The outer layer aids in the comfort and removal of the tampon from a body cavity.

Brief Description of the Drawings

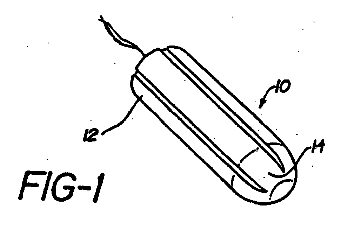

Fig. 1 is a perspective view of a tampon according to the present invention with a cover layer substantially covering an absorbent structure.

Detailed Description of the Invention

As used herein the specification and the claims, the term "Absorbent tampon" and variations thereof, includes nasal tampons, bandages, and vaginal tampons.

"Ease of removal" describes how difficult it is for a user to remove a tampon from the body cavity- how hard the user has to pull on the string to get the tampon out. In light flow situations, it is typical that the user requires more strength to remove the tampon.

"Comfort" refers to the physical feeling a user will experience during removal of a tampon, A negative feeling of comfort (or discomfort) can include pain. Users often describe their experience as the drier they are, the more painful it is when withdrawing the tampon from the body cavity. A tampon that is more difficult to remove may additionally be more uncomfortable but not all the time.

"Shedding" refers to fibers that may be left behind when removing a conventional tampon during a dry/low flow situation.

Absorbent tampons are usually substantially cylindrical masses of compressed absorbent material having a central axis and a radius that defines the outer circumferential surface of the tampon. Tampons are often formed by first obtaining a shaped mass of absorbent material called a tampon blank. This blank can be in the form of a roll of sheet-like material, a segment of a continuous absorbent material, a mass of randomly or substantially uniformly oriented absorbent material, an individually prepared or cast mass of absorbent material, and the like.

The tampon blank is relatively uncompressed and has a relatively low density. It is then compressed to form a product having overall dimensions less than those of the blank prior to use. The compressed tampons may have a generally uniform density throughout the tampon, or they may have regions of differing density as described in the commonly assigned applications to Friese et al., U.S. Serial No. 07/596,454, and Leutwyler et al., US Pat. No. 5,813,102. Tampons also usually include a cover or some other surface treatment and a withdrawal string or other removal mechanism.

The present invention pertains to an absorbent tampon, such as catamenial tampons. In particular, the invention pertains to a tampon that is easy to insert and remove. The tampon has an absorbent core and a cover. The cover incorporates a combination of bicomponent materials and rayon that has been grafted with CMC.

The tampon blank is substantially enclosed by a fluid-permeable cover. Thus, the cover encloses a majority of the outer surface of the tampon. This may be achieved as disclosed in Friese, US Pat. No. 4,816,100. In addition, either or both ends of the tampon may be enclosed by the cover. Of course, for processing or other reasons, some portions of the surface of the tampon may be free of the cover. For example, the insertion end of the tampon and a portion of the cylindrical surface adjacent this end may be exposed, without the cover to allow the tampon to more readily accept fluids.

The modified cellulosic fibers have carboxyalkyl substituted cellulosic regions. The modified cellulosic fibers may be derived from regenerated cellulosic fiber or natural cellulosic fibers. A useful, non-limiting list of useful such cellulosic fibers includes natural fibers such as cotton, wood pulp, jute, hemp, and the like; and processed fibers such as regenerated cellulose, cellulose nitrate, cellulose acetate, and rayon. Preferably, the modified cellulosic fibers are derived from cotton or rayon. The cellulosic fibers are modified to have carboxyalkyl substituted cellulosic regions, for example by the solvent-spun process disclosed in US Pat No. 5,731,083.

Rayon is a cellulosic fiber commonly used as a nonwoven absorbent fiber. Cellulose is a polysaccharide $(C_6H_{10}O_5)_x$ of glucose units. The degree of substitution (D.S.) indicates the number of substituent groups per glucose unit in the cellulose molecular chain. Since there are originally three hydroxyl groups and hence three possible points of substitution per glucose unit, the maximum degree of substitution is 3.

In one embodiment of the present invention, carboxymethyl cellulose (CMC) is reacted with a hydroxyl group of the glucose unit of rayon. Water is formed and the CMC radical is attached to the glucose unit. A preferred range of substitution of the glucose unit is 0.1 to 0.5. For a 0.35 degree of substitution, the average of 0.35 carboxymethyl radical is substituted onto one glucose unit. A more preferred range is 0.2 to 0.5 and a most preferred range is 0.3 - 0.5. Cellulosic fibers having a DS of at least 0.1 is disclosed in US Pat. No. 5,731,083.

In a preferred embodiment of the present invention, the CMC substituted rayon is then blended with fiber to form a nonwoven layer. The fiber may be any commercially available fiber including but not limited to natural fibers, synthetic fibers, bicomponent fibers or combination thereof. In particular, it is preferred that the fibers be thermoplastic such as polyethylene (PE), polyacrylic, polyvinyl acetate (PVA) and polypropylene (PP). While any bicomponent fibers known to those skilled in the art may be used, examples of suitable bicomponent fibers include PE/polyester (PET), PE/PP, PET/PET and PE/nylon. In particular, bicomponent fibers such as polyester/polyethylene and polypropylene/polyethylene are preferred.

Methods of cover bonding include, but are not limited to, powder bonding, through-air bonding, emboss-calender, adhesive bonding, and needlepunching. The ratio of CMC substituted rayon to bicomponent fibers affects the ability of the cover or wrapper to gain sufficient strength and to adhere to the absorbent core, especially if the cover is thermal bonded. For example, too high a concentration of CMC substituted rayon fibers will result in a weak cover and poor adherence to an absorbent core made from rayon and cotton.

In the present invention, it is preferred that the CMC substituted rayon be at least 5% but not more than 40% of the overall composition of the cover or wrapper. The amount of bicomponent present in the cover or wrapper is therefore preferred to be in the range of 60% to 95%. A more preferred amount of bicomponent present in the cover is 70% to 90% while the most preferred range is 80% to 85%.

When exposed to fluid or body moisture, the cover has a lubricious feeling. While not being bound to any theory, it is believed that this lubriciousness aids in the removal of a tampon after use. The tampon becomes wet from body fluids and the cover becomes lubricious. Less force is required to remove the tampon from the body cavity and there is less friction on the body cavity walls. The cover also may prevent vaginal drying by the absorbent portion of the tampon, especially highly absorbent tampons. A measure of this ability of the cover material to retain moisture and provide lubricity is the Centrifuge Retention (distilled water), as described below in the Examples. The Centrifuge Retention (distilled, water) is at least about 1 g/g for the cover, more preferably, at least about 5 g/g, and most preferably, the cover has a Centrifuge Retention (distilled water) of at least about 8 g/g.

It is preferred that the basis weight of the final material be between 6 to 20 gsm. A more preferred basis weight range is 8 to 12 gsm with the most preferred range being 8-9 gsm. The cover of the present invention can ease the withdrawal of the tampon from the body cavity.

Fig. 1 shows a tampon 10 having a cover layer or cover 12 substantially enclosing the absorbent structure 14. A withdrawal string 16 extends from the base or withdrawal end of the tampon. The following description is a brief example of how such a cover may be attached to an absorbent core to form a tampon blank. Alternately, other methods known to those skilled in the art may be used to attach the cover to the absorbent core.

As described in greater detail in Friese et al., US Pat. No. 4,816,100, a cover strip can be laid upon and sealed to nonwoven absorbent ribbon. The withdrawal string is placed around the ribbon, and the absorbent ribbon is wound upon itself to form a tampon blank. The finished tampon blank can then be compressed to its final form, e.g., as described in Leutwyler et al., US Pat. No. 5,911,712.

Absorbent materials useful in the formation of the absorbent body include fiber, foam, superabsorbent, hydrogels, wood pulp, and the like. Preferred absorbent material for the present invention includes foam and fiber. Absorbent foams may include hydrophilic foams, foams which are readily wetted by aqueous fluids as well as foams in which the cell walls that form the foam themselves absorb fluid.

Fibers employed in the formation of the absorbent body may include regenerated cellulosic fiber, natural fibers and synthetic fibers. Preferably, the materials employed in the formation of a vaginal tampon according to the present invention include fiber, foam, hydrogels, wood pulp, and the like, but are essentially superabsorbent-free. Thus, the absorbent structure have a relatively low Centrifuge Retention (distilled water). The Centrifuge Retention (distilled water) is less than about 100 g/g for the absorbent structure, more preferably, less than about 10 g/g, and most preferably, the absorbent structure has a Centrifuge Retention (distilled water) of at less than about 8 g/g.

A useful, non-limiting list of useful absorbent body fibers includes natural fibers such as cotton, wood pulp, jute, and the like; and processed fibers such as regenerated cellulose, cellulose nitrate, cellulose acetate, rayon, polyester, polyvinyl alcohol, polyolefin, polyamine, polyamide, polyacrylonitrile, and the like. Other fibers in addition to the above fibers may be included to add desirable characteristics to the absorbent body. Preferably, tampon fibers are rayon or cotton, and more preferably, the fibers are rayon. The fibers may have any useful cross-section.

Fiber cross-sections include multi-limbed and non-limbed. Multi-limbed, regenerated cellulosic fibers have been commercially available for a number of years. These fibers are known to possess increased specific absorbency over non-limbed fibers. Commercial example of these fibers is the Danufil VY multilimbed viscose rayon fibers available from Acordis UK Ltd., Spondon, England. These fibers are described in detail in Wilkes et al., US Pat. No. 5,458,835.

Tampons are generally categorized in two classes: applicator tampons and digital tampons. Applicator tampons use a relatively rigid device to contain and protect the tampon prior to use. To insert the tampon into a body cavity, the applicator is partially inserted into the body cavity, and the tampon can be expelled therefrom. Because the tampon is protected by the rigid applicator device, the tampon need not have a high degree of dimensional stability. In contrast, digital tampons do not have an applicator to help guide them into the body cavity and require sufficient stability to allow insertion without using an applicator. The tampon of this invention may be either a digital tampon or one that requires an applicator to insert it the body cavity.

## Examples

The present invention will be further understood by reference to the following specific Examples that are illustrative of specific elements of the present invention. It is to be understood that many variations of composition, form and method of producing the invention would be apparent to those skilled in the art. The following Examples, wherein parts and percentages are by weight unless otherwise indicated, are only illustrative.

## Example 1

Cover material samples were made generally according to the following method: Bicomponent fusible and non-fusible fiber were blended and carded to form a web. The web of the fiber blend was then passed through a calender section equipped with a pattern steel roll and a smooth steel roll. The surface temperature of emboss-calender rolls is set above the melting temperature of the bicomponent sheath that is usually around 130° C. The fabric was then slit and prepared for determining their tensile strength. Three to five samples were removed from the fabric and oriented in either the machine direction ("MD") or cross direction ("CD"), depending upon the direction to be measured. A notch was made at approximately the center of the length of the sample to form a test width of 45 mm for MD tensile and 50 mm for CD tensile. Each end of the sample was then secured in the jaws of an Instron, and the tester was set to move the jaws that had an initial distance of 10 cm, apart at a rate of 20 cm/min. The force at which the sample failed was recorded at the sample strength.

Control fabric - cover fabric material made from 100% 1071 PET/PE bicomponent fiber from IPI, Morristown, TN.

Sample 1 fabric -10% by weight of Hydrocel™ CMC fiber from Acordis, Spondon, UK with 0.25-0.3 degree of substitution was first blended with 90% of 1071 PET/PE bicomponent fiber from IPI, Morristown, TN to form cover fabric material.

Sample 2 fabric - 15% by weight of Hydrocel™ CMC fiber from Acordis, Spondon, UK with 0.25-0.3 degree of substitution was first blended with 85% of 1071 PET/PE bicomponent fiber from IPI, Morristown, TN to form cover fabric material.

**TABLE 1**

|  | MD Tensile (N/45 mm) | MD elongation (%) | CD Tensile (N/50 mm) |
|---|---|---|---|
| Control with 100% 1071 PE/PET Bicomcomponent fiber | 14 | 15 | 0.8 |
| Sample 1 | 13.7 | 17 | 2.3 |

The Sample 2 fabric produced similar results: These data show that a fabric made with CMC substituted rayon had equivalent properties as control fabric. The physical properties are important because without adequate strength, especially MD tensile, the cover could not be made and tampon could not be converted in current commercial processing equipment.

## Example 2

Several materials were tested to determine their Centrifuge Retention according the following test:
The following apparatus are used to determine water centrifuge retention:

- centrifuge having a 9 inch (22.9 cm) diameter capable of rotation at 1400 rpm
- balance, accurate to $\pm 0.05$ grams or better,
- Dexter type 1234T9 or similar material for preparation of tea bags,
- Clicker Press to form "tea bags" from the Dexter material,
- container large enough to immerse and submerge the tea bag samples,
- support clamps/stands, and
- drainage pan.

A tea bag large enough to contain a 0.5 g sample was made, and the sample material (pre-weighed) was placed into the tea bag, which was then sealed. An identical size bag should also be made for a blank.
The dry sample and blank bags were weighed and then placed into a container with distilled water to allow free-swelling for 30 minutes. After soaking for 30 minutes, the sample and blank bags were taken out of the bath and hung vertically on the support stand clips for 10 minutes. The wet sample bags and blanks were then weighed. Next, the sample and blank bags were evenly distributed in the centrifuge which was operated at 1400 rpm for 5 minutes. The weight of the blank and sample bags was again weighed.

$$\text{Centrifuge Retention, g/g} = C - B - A / A$$

Where:

A = dry weight of test sample
B = centrifuge weight of blank bag
C = centrifuge weight of sample bag

Five samples of each material were tested according to the test procedure outlined above. The results are illustrated in TABLE 2, below.

**TABLE 2**

| Sample | (A) Dry weight of sample | (C) Wet wt. of sample and tea bag, g | (D) Centrifuge wt. of sample + tea bag, g | (F) Wet wt. of tea bag, g | (G) Centrif. wt. of tea bag, g | Free Swell Absorb. g/g | Centrif. Retent. g/g |
|---|---|---|---|---|---|---|---|
| Sample 1 | 0.50 | 11.46 | 5.61 | 0.56 | 0.19 | 20.74 | 9.80 |
| of Ex. 1 | 0.50 | 11.87 | 5.50 | 0.55 | 0.16 | 21.58 | 9.66 |
|  | 0.50 | 12.85 | 5.37 | 0.53 | 0.21 | 23.67 | 9.32 |

(continued)

| Sample | (A) Dry weight of sample | (C) Wet wt. of sample and tea bag, g | (D) Centrifuge wt. of sample + tea bag, g | (F) Wet wt. of tea bag, g | (G) Centrif. wt. of tea bag, g | Free Swell Absorb. g/g | Centrif. Retent. g/g |
|---|---|---|---|---|---|---|---|
| | 0.50 | 11.85 | 5.18 | 0.49 | 0.20 | 21.71 | 8.96 |
| | 0.50 | 14.85 | 5.19 | 0.57 | 0.20 | 27.53 | 8.96 |
| **AVG.** | **0.50** | **12.58** | **5.37** | **0.54** | **0.19** | **23.05** | **9.34** |
| | | | | | | | |
| Sample 3 | 0.50 | 14.98 | 1.11 | 0.39 | 0.14 | 28.16 | 0.94 |
| o.b ® Regular Flushable Applica-tor Tampon | 0.51 | 14.39 | 1.10 | 0.47 | 0.17 | 26.51 | 0.84 |
| | 0.50 | 13.83 | 1.30 | 0.53 | 0.19 | 25.55 | 1.23 |
| | 0.50 | 14.92 | 1.21 | 0.43 | 0.17 | 27.90 | 1.09 |
| | 0.50 | 14.49 | 1.05 | 0.46 | 0.16 | 27.07 | 0.78 |
| **AVG.** | **0.50** | **14.52** | **1.15** | **0.46** | **0.17** | **27.04** | **0.98** |
| | | | | | | | |
| Sample 4 | 0.50 | 15.48 | 1.19 | 0.48 | 0.18 | 28.95 | 1.01 |
| Tampax® Satin Regular (Multi-pax) | 0.50 | 14.60 | 1.14 | 0.48 | 0.20 | 27.23 | 0.88 |
| | 0.50 | 15.53 | 1.14 | 0.45 | 0.16 | 29.15 | 0.96 |
| | 0.50 | 15.40 | 1.06 | 0.42 | 0.23 | 28.96 | 0.66 |
| | 0.50 | 14.66 | 1.16 | 0.45 | 0.18 | 27.42 | 0.96 |
| **AVG.** | **0.50** | **15.13** | **1.14** | **0.46** | **0.19** | **28.34** | **0.89** |
| | | | | | | | |
| Sample 5 | 0.50 | 11.31 | 1.01 | 0.46 | 0.21 | 20.72 | 0.61 |
| Kotex ® Security Super Plus | 0.50 | 12.57 | 1.04 | 0.47 | 0.28 | 23.14 | 0.53 |
| | 0.50 | 11.33 | 1.00 | 0.47 | 0.25 | 20.70 | 0.49 |
| | 0.50 | 12.62 | 1.10 | 0.53 | 0.23 | 23.24 | 0.74 |
| | 0.50 | 12.48 | 1.05 | 0.59 | 0.26 | 22.83 | 0.58 |
| **AVG.** | **0.50** | **12.06** | **1.04** | **0.50** | **0.25** | **22.12** | **0.59** |
| | | | | | | | |
| Sample 6 | 0.50 | 12.78 | 1.13 | 0.57 | 0.27 | 23.40 | 0.72 |

(continued)

| Sample | (A) Dry weight of sample | (C) Wet wt. of sample and tea bag, g | (D) Centrifuge wt. of sample + tea bag, g | (F) Wet wt. of tea bag, g | (G) Centrif. wt. of tea bag, g | Free Swell Absorb. g/g | Centrif. Retent. g/g |
|---|---|---|---|---|---|---|---|
| Playtex® Gentle Glide Super | 0.50 | 13.60 | 0.99 | 0.51 | 0.12 | 25.14 | 0.73 |
|  | 0.50 | 12.66 | 0.96 | 0.53 | 0.12 | 23.23 | 0.66 |
|  | 0.50 | 13.22 | 0.95 | 0.54 | 0.12 | 24.33 | 0.66 |
|  | 0.50 | 13.03 | 0.94 | 0.49 | 0.16 | 24.04 | 0.56 |
| AVG. | 0.50 | 13.06 | 0.99 | 0.53 | 0.16 | 24.03 | 0.67 |
|  |  |  |  |  |  |  |  |
| Sample 7 | 0.10 | 25.56 | 14.51 | 1.44 | 0.83 | 239.74 | 135.57 |
|  | 0.10 | 23.60 | 13.14 | 1.42 | 0.87 | 219.68 | 121.07 |
|  | 0.10 | 23.46 | 13.33 | 1,46 | 0.86 | 218.13 | 123.25 |
| AVG. | 0.10 | 24.21 | 13.66 | 1.44 | 0.85 | 225.85 | 126.63 |

*Fluff pulp structure containing about 25 wt-% superabsorbent, available from Rayonier. Due to bag breakage, only three samples of 0.10 g were used.

These data show that a cover with modified cellulosic fibers has a Centrifuge Retention (distilled water) of about 9 g/g, while a superabsorbent-containing pulp structure has a Centrifuge Retention (distilled water) of greater than 100 g/g and commercial tampon structures have a Centrifuge Retention (distilled water) of about 1 g/g or less.

**Claims**

1. An absorbent tampon comprising

a) an absorbent structure comprising an absorbent component that consists essentially of absorbent material that has a Centrifuge Retention (distilled water) of less than about 10 g/g;
b) an outer layer that substantially covers the absorbent structure and that has a different composition than the absorbent structure, comprising modified cellulosic fibers that have carboxyalkyl substituted regions and a Centrifuge Retention (distilled water) of at least about 1 g/g,

wherein the Centrifuge Retention is determined with a centrifuge having a 22,9 cm (9 inch) diameter capable of rotation at 1.400 rpm and which is calculated according to the following formula

$$\text{Centrifuge Retention (g/g)} = (C - B - A)/A,$$

wherein
A = dry weight of test sample, B = centrifuge weight of blank bag and C = centrifuge weight of sample bag, and wherein the blank bag and the sample bag are placed in distilled water to allow free-swelling for 30 minutes whereafter the blank bag and the sample bag are hung vertically for 10 minutes prior to weighing, whereupon the sample bag and the blank bag are centrifuged at 1.400 rpm for 5 minutes, and whereupon the weight of the blank bag and the sample bag were determined.

2. The absorbent tampon according to claim 1 wherein the absorbent structure is substantially free of said modified cellulosic fibers.

3. The absorbent tampon of claim 1 or 2 wherein the outer layer that substantially covers the absorbent structure comprises a non-woven fabric comprising a mixture of said modified cellulosic fibers and thermoplastic fibers.

4. The absorbent tampon of claim 3 wherein the thermoplastic fibers are formed of polymeric materials selected from the group consisting of polyolefins, polyesters, polyamides, polyamines, and combinations thereof.

5. The absorbent tampon of claim 4 wherein the thermoplastic fibers comprise bicomponent fibers.

6. The absorbent tampon of claim 5 wherein the bicomponent fiber is a polyester/polyethylene or a polypropylene/polyethylene fiber.

7. The absorbent tampon according to any of claims 3 to 6 wherein the outer layer that substantially covers the absorbent structure comprises about 5 to about 40 wt.-% modified cellulosic fibers and about 95 to about 60 wt.-% thermoplastic fibers.

8. The absorbent tampon of any of the proceeding claims wherein the modified cellulosic fibers comprise rayon fibers having carboxymethyl cellulose ("CMC") regions on their outer surface.

9. The absorbent tampon of claim 8 wherein the CMC is substituted onto rayon with a degree of substitution of about 0.2 to about 0.5 CMC groups per glucose unit.

10. The absorbent tampon according to any of claims 5 to 9 wherein the outer layer that substantially covers the absorbent structure comprises a mixture of CMC substituted rayon and a bicomponent fiber.

11. The absorbent tampon according to any of the proceeding claims wherein the outer layer that substantially covers the absorbent structure is bonded to said absorbent structure.


**Patentansprüche**

1. Absorbierender Tampon, umfassend:

a) eine absorbierende Struktur, die einen absorbierenden Bestandteil umfasst, der im Wesentlichen aus absorbierendem Material besteht, das ein Zentrifugen-Rückhaltevermögen (destilliertes Wasser) von weniger als zirka 10 g/g aufweist;
b) eine äußere Lage, welche die absorbierende Struktur im Wesentlichen bedeckt und eine andere Zusammensetzung als die absorbierende Struktur aufweist und modifizierte Zellulosefasern umfasst, die Carboxyalkyl-substituierte Bereiche aufweisen, und welche ein Zentrifugen-Rückhaltevermögen (destilliertes Wasser) von mindestens zirka 1 g/g aufweist,

wobei das Zentrifugen-Rückhaltevermögen mit einer Zentrifuge mit einem Durchmesser von 22,9 cm (9 Zoll) bestimmt wird, die zu einer Rotation mit 1.400 Umdrehungen pro Minute in der Lage ist, und gemäß der folgenden Formel berechnet wird:

$$\text{Zentrifugen-Rückhaltevermögen (g/g)} = (C-B-A)/A,$$

wobei
A = Trockengewicht der Testprobe, B = Zentrifugengewicht des leeren Beutels und C = Zentrifugengewicht des Proben-Beutels, und wobei der leere Beutel und der Proben-Beutel in destilliertem Wasser platziert werden, um diese 30 Minuten lang frei schwellen zu lassen, wonach der leere Beutel und der Proben-Beutel vor dem Wiegen 10 Minuten lang vertikal aufgehängt werden, woraufhin der Proben-Beutel und der leere Beutel mit 1.400 Umdrehungen pro Minute 5 Minuten lang zentrifugiert werden und woraufhin das Gewicht des leeren Beutels und das Gewicht des Proben-Beutels bestimmt werden.

2. Absorbierender Tampon nach Anspruch 1, **dadurch gekennzeichnet, dass** die absorbierende Struktur im Wesentlichen frei von den modifizierten Zellulosefasern ist.

3. Absorbierender Tampon nach Anspruch 1 oder 2, **dadurch gekennzeichnet, dass** die äußere Lage, welche die absorbierende Struktur im Wesentlichen bedeckt, ein Vliesgewebe umfasst, das ein Gemisch aus den modifizierten Zellulosefasern und thermoplastischen Fasern umfasst.

4. Absorbierender Tampon nach Anspruch 3, **dadurch gekennzeichnet, dass** die thermoplastischen Fasern aus polymeren Materialien, ausgewählt aus der Gruppe bestehend aus Polyolefinen, Polyestern, Polyamiden, Polyaminen und Kombinationen derselben, gebildet sind.

5. Absorbierender Tampon nach Anspruch 4, **dadurch gekennzeichnet, dass** die thermoplastischen Fasern Zweikomponentenfasern umfassen.

6. Absorbierender Tampon nach Anspruch 5, **dadurch gekennzeichnet, dass** die Zweikomponentenfaser eine Polyester/Polyethylen-Faser oder eine Polypropylen/Polyethylen-Faser ist.

7. Absorbierender Tampon nach einem der Ansprüche 3 bis 6, **dadurch gekennzeichnet, dass** die äußere Lage, welche die absorbierende Struktur im Wesentlichen bedeckt, zirka 5 bis zirka 40 Gew.-% modifizierte Zellulosefasern und zirka 95 bis zirka 60 Gew.-% thermoplastische Fasern umfasst.

8. Absorbierender Tampon nach einem der vorangehenden Ansprüche, **dadurch gekennzeichnet, dass** die modifizierten Zellulosefasern Rayonfasern umfassen, die Carboxylmethylzellulose-Bereiche ("CMC") auf ihrer äußeren Oberfläche aufweisen.

9. Absorbierender Tampon nach Anspruch 8, **dadurch gekennzeichnet, dass** die CMC auf Rayon mit einem Substitutionsgrad von zirka 0,2 bis zirka 0,5 CMC-Gruppen pro Glukoseeinheit substituiert ist.

10. Absorbierender Tampon nach einem der Ansprüche 5 bis 9, **dadurch gekennzeichnet, dass** die äußere Lage, welche die absorbierende Struktur im Wesentlichen bedeckt, ein Gemisch aus mit CMC substituiertem Rayon und einer Zweikomponentenfaser umfasst.

11. Absorbierender Tampon nach einem der vorangehenden Ansprüche, **dadurch gekennzeichnet, dass** die äußere Lage, welche die absorbierende Struktur im Wesentlichen bedeckt, mit der absorbierenden Struktur verbunden ist.

**Revendications**

1. Tampon absorbant, comportant :

   a) une structure absorbante comportant un composant absorbant situé essentiellement d'un matériau absorbant ayant une rétention centrifuge (eau distillée) inférieure à environ 10 g/g ;
   b) une couche extérieure qui recouvre sensiblement la structure absorbante et qui a une composition différente par rapport à la structure absorbante, comportant des fibres cellulosiques modifiées présentant des régions substituées en carboxyalkyle et une rétention centrifuge (eau distillée) d'au moins environ 1 g/g,

   dans lequel la rétention centrifuge est déterminée en utilisant un dispositif centrifuge d'un diamètre de 22,9 cm (9 pouces) capable de tourner à 1400 tpm, et est calculée selon la formule suivante

$$\text{rétention centrifuge (g/g)} = (C - B - A)/A,$$

   où
   A = poids à sec de l'échantillon de test, B = poids centrifuge du sac vide, et C = poids centrifuge du sac à échantillon, et dans laquelle le sac vide et le sac à échantillon sont placés dans de l'eau distillée pendant 30 minutes afin de permettre leur libre gonflement, après quoi le sac vide et le sac à échantillon sont suspendus verticalement pendant 10 minutes avant pesée, suite à quoi le sac à échantillon et le sac vide sont centrifugés à 1400 tpm pendant 5 minutes, puis le poids du sac vide et du sac à échantillon sont alors déterminés.

2. Tampon absorbant selon la revendication 1, dans lequel la structure absorbante est sensiblement exempte desdites fibres cellulosiques modifiées.

3. Tampon absorbant selon la revendication 1 ou 2, dans lequel la couche extérieure qui recouvre sensiblement la structure absorbante comporte un tissu non tissé comprenant un mélange desdites fibres cellulosiques modifiées et de fibres thermoplastiques.

4. Tampon absorbant selon la revendication 3, dans lequel les fibres thermoplastiques sont formées de matériaux polymères choisis dans le groupe constitué de polyoléfines, de polyesters, de polyamides, de polyamines, et de combinaisons de ceux-ci.

5. Tampon absorbant selon la revendication 4, dans lequel les fibres thermoplastiques comportent des fibres bicomposants.

6. Tampon absorbant selon la revendication 5, dans lequel la fibre bicomposant est une fibre de polyester/polyéthylène ou polypropylène/polyéthylène.

7. Tampon absorbant selon l'une quelconque des revendications 3 à 6, dans lequel la couche extérieure qui recouvre sensiblement la structure absorbante comporte environ 5 à environ 40 % en poids de fibres cellulosiques modifiées et environ 95 à environ 60 % en poids de fibres thermoplastiques.

8. Tampon absorbant selon l'une quelconque des revendications précédentes, dans lequel les fibres cellulosiques modifiées comportent des fibres de rayonne ayant des régions de carboxyméthylcellulose ("CMC") sur leur surface extérieure.

9. Tampon absorbant selon la revendication 8, dans lequel la CMC est substituée sur de la rayonne avec un degré de substitution d'environ 0,2 à environ 0,5 groupe CMC par unité de glucose.

10. Tampon absorbant selon l'une quelconque des revendications 5 à 9, dans lequel la couche extérieure qui recouvre sensiblement la structure absorbante comporte un mélange de rayonne substituée par de la CMC, et une fibre bicomposant.

11. Tampon absorbant selon l'une quelconque des revendications précédentes, dans lequel la couche extérieure qui recouvre sensiblement la structure absorbante est collée sur ladite structure absorbante.

FIG-1

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- US 3005456 A, Graham
- US 3371666 A, Lewing
- US 5731083 A, Bahia
- US 3683912 A, Olson
- WO 9002542 A, Snider
- US 4056103 A, Kaczmarzyk
- WO 9932061 A1
- GB 975872 A
- US 3390671 A
- US 07596454 B, Friese
- US 5813102 A, Leutwyler
- US 4816100 A, Friese
- US 5911712 A, Leutwyler
- US 5458835 A, Wilkes